# EUROPÄISCHE PATENTANMELDUNG

(11) **EP 1 995 316 A1**
(43) Veröffentlichungstag der Anmeldung: **26.11.2008**
(21) Anmeldenummer: 07010424.5
(22) Anmeldetag: 25.05.2007
(51) Int. Cl.: C12N 15/10, C12N 15/87

(54) **Verfahren zur schonenden Zellaufreinigung, Zellgewinnung und Transfektion von Zellen**

(71) Anmelder: Qiagen GmbH, 40724 Hilden (DE)
(72) Erfinder: Andreou, Ioanna, 50674 Köln (DE); Janosch, Andrea, 40589 Düsseldorf (DE); Himmelreich, Ralf, 40764 Langenfeld (DE)
(74) Vertreter: Polypatent

(57) **Zusammenfassung**

Die vorliegende Erfindung betrifft ein Verfahren zur Zellaufreinigung, zur Zellgewinnung aus Kulturen und zur Transfektion der Zellen unter besonders schonenden Bedingungen, wobei dieses Verfahren zusätzlich gekoppelt werden kann mit einer nachfolgenden Isolierung und Aufreinigung von Polynucleotiden aus den gegebenenfalls transfizierten Zellen, sowie einen Kit und eine Vorrichtung zur Durchführung dieses Verfahrens.

## Beschreibung

Die vorliegende Erfindung betrifft ein Verfahren zur Zellaufreinigung, zur Zellgewinnung aus Kulturen und/oder zur Transfektion von Zellen unter besonders schonenden Bedingungen, wobei dieses Verfahren zusätzlich gekoppelt werden kann mit einer nachfolgenden Isolierung und Aufreinigung von Polynucleotiden aus den gegebenenfalls transfizierten Zellen, sowie einen Kit und eine Vorrichtung zur Durchführung dieses Verfahrens.

Bisherige Verfahren zur Zellgewinnung oder Aufreinigung von Zellen umfassen üblicherweise einen Zentrifugationsschritt, durch den die Zellen aus einem Suspensionsmedium durch Zentrifugalkraft abgetrennt werden. Dieser Zentrifugationsschritt muss unter Bedingungen durchgeführt werden, die keine nachteilige Auswirkung auf die Zellen haben. Beispielsweise führt eine zu hohe Zentrifugalkraft zur Zerstörung der Zellen. Außerdem werden hier nicht alle Zellen genommen, viele verbleiben in Suspension. Da bei einer Zentrifugation die Zellen mehrmals aus der sterilen Werkbank entfernt werden müssen, werden sie zusätzlich der Gefahr ausgesetzt kontaminiert zu werden und das ist ein sehr kritischer Punkt bei Zell-Experimenten.

Eine weitere Möglichkeit, Zellen aus flüssigen Medien zu entfernen, ist die Filtration, wobei auch hier Bedingungen eingehalten werden müssen, die nicht zu einer Zerstörung der Zellen führen. Darüber hinaus ist der Zellverlust bei Filtrationsvorrichtungen relativ hoch, da die Zellen anschließend nur schwer von den Filtermaterialien entfernt werden können.

Ein ebenfalls häufig angewendetes Verfahren, insbesondere für eine selektive spezifische Zellaufreinigung oder -gewinnung ist das Binden von Zellen an feste Oberflächen über spezifische Moleküle, die Oberflächenstrukturen oder Oberflächenproteine dieser spezifischen Zellen "erkennen", beispielsweise die Bindung über spezifische Rezeptoren oder Oberflächenproteine der Zellen.

Die Verwendung von magnetischen Kügelchen, sog. "Beads" für die Aufreinigung von Polynucleotiden aus flüssigen Medien ist seit längerem bekannt. Beispielsweise beschreibt die EP-A 515 484, die EP-A 764 206 und die WO 01/71732 jeweils ein Verfahren zur Gewinnung von Nucleinsäuren aus einem flüssigen Medium, wobei diesem Medium magnetische Kügelchen zugesetzt werden, an die die Nucleinsäuremoleküle aufgrund unspezifischer Bindungen gebunden werden, oder sich daran anlagern. In allen drei Dokumenten wird jedoch eine Zell-Lyse vor Zugabe der magnetischen Kügelchen beschrieben, was einer Zellgewinnung oder -aufreinigung durch die Kügelchen entgegensteht, bzw. eine Zellgewinnung durch Zentrifugation vor der Aufreinigung der Nucleinsäuren, wobei erst letztere mit Hilfe der magnetischen Kügelchen erfolgt. Eine Anlagerung von intakten Zellen an die magnetischen Kügelchen wird im Stand der Technik nicht erwähnt.

Die objektive Aufgabe der vorliegenden Erfindung war es, ein einfaches, schonendes und effektives Verfahren zur Zellgewinnung, Zellaufreinigung und/oder Transfektion von Zellen bereitzustellen, das darüber hinaus ohne Zentrifugationsschritte eine Isolierung von Polynucleinsäuren aus diesen Zellen erlaubt. Diese Aufgabe wird gelöst durch ein Verfahren zur Zellaufreinigung und/oder Zellgewinnung und/oder Transfektion von Zellen, welches die Zugabe von magnetischen Kügelchen zu intakten Zellen umfasst, wobei die magnetischen Kügelchen mit einer Glas- oder Polymerbeschichtung überzogen sind und auf ihrer Oberfläche chemische Gruppen tragen, die eine unspezifische Bindung der Zellen an die Oberfläche erlaubt.

Kügelchen, die für das vorliegende Verfahren geeignet sind, können jeden Typ von bisher bekannten magnetischen Kügelchen umfassen, bei denen ein magnetischer Kern mit einer Glas- oder Polymerbeschichtung überzogen ist, und auf ihrer Oberfläche Gruppen tragen, die eine unspezifische Anlagerung oder Bindung von intakten Zellen an die Kügelchen ermöglichen. Bevorzugt können solche Kügelchen eingesetzt werden, die auf ihrer Oberfläche Säuregruppen tragen, bevorzugt Carbonsäuregruppen, Phosphorsäuregruppen oder Schwefelsäuregruppen oder deren Salze, insbesondere bevorzugt Carbonsäuregruppen oder deren Salze, wobei die genannten Gruppen unmittelbar an die Oberfläche gebunden sein können, oder Teil des die Oberflächenbeschichtung bildenden Polymers sein können, über Spacer-Moleküle an die Oberfläche gebunden sein können, oder Teile einer Verbindung sein können, die an die Oberfläche der Kügelchen gebunden sind. In einer bevorzugten Ausführungsform tragen die Kügelchen auf ihrer Oberfläche eine insgesamt schwach negative Gesamtladung, da bei Vorliegen solcher Bedingungen die Zellen besonders wirksam gebunden werden. Eine neutrale bis schwach positive Ladung der Kügelchen ist ebenfalls möglich, wenn auch für das vorliegende Verfahren nicht bevorzugt. Beispiele für geeignete carboxylierte Polymere, die als Beschichtungsmaterial für die Kügelchen geeignet sind und eine für die Erfindung geeignete Oberfläche bereitstellen sind in der gleichzeitig anhängigen deutschen Patentanmeldung DE 10 2005 040 259.3 (Qiagen, Anmeldetag 24.8.2005) im Detail beschrieben. Beispiele für Verbindungen, die an die Oberfläche gebunden sein können, sind Glycin, Hydrazin, Asparaginsäure, 6-Aminocapronsäure, NTA (Nitrilotriacetic acid), PEI (Polyethylenimine), Polyacrylsäure (PAA), HCl, Glycerin, Diglyme (Diethylene Glycol Dimethyl Ether Formel: (CH₃OCH₂CH₂)₂O, glyme : Glycol diethers ), Pentaerythritol, Toluol, Polyallylamin, Jeffamin 500 (O,O-Bis(2-aminopropyl)-polyethylenglycol 500), Polyethylen-hexamin, Polyethylenimin, Bis-Tris (Bis(2-hydroxyethyl)iminotris(hydroxymethyl)methane), DIPEA (N,N'-Düsopropylethylamine), oder Kombinationen davon, ohne auf diese beschränkt zu sein. Ebenfalls bevorzugte magnetische Kügelchen sind solche, wie sie in der gleichzeitig anhängigen deutschen Patentanmeldung DE 10 2005 058 979.9 (Qiagen, Anmeldetag 9.12.2005) beschrieben sind. Solche geeigneten magnetischen Kügelchen sind auf dem Markt erhältlich.

Die Zellen, die über das Verfahren der vorliegenden Erfindung aus Medien gewonnen werden können, sind bevorzugt eukaryontische Zellen, insbesondere Zellen, die häufig für Transfektionsexperimente verwendet werden, wie z. B. Säugetierzellen oder Insektenzellen, welche als Suspensionskulturen oder adhärente Kulturen geführt werden können, Zellen aus Blut oder Gewebe, die in gemischten oder reinen Kulturen vorliegen können, oder nach Isolierung in flüssigen Medien aufgenommen wurden. Eine Zellisolierung aus Vollblut oder Blutserum ist ebenfalls möglich, in allen genannten Fällen ist jedoch die Zellgewinnung oder -aufreinigung aus einer Suspension der Zellen in flüssigem Medium bevorzugt.

Die Zellen lagern sich bei Kontakt mit den magnetischen Kügelchen über unspezifische Wechselwirkungen mit deren Oberfläche an die Kügelchen an. Hierbei kommen mechanische oder elektrostatische Wechselwirkungen in Betracht, ohne dass die Erfindung durch diese theoretischen Betrachtungen beschränkt sein soll. Die Größe der Kügelchen im Vergleich zu den Zellen spielt für die Zellgewinnung / - reinigung keine wesentliche Rolle, jedoch ist es insbesondere im Falle einer nachfolgenden Transfektion, wie es unten beschrieben ist, bevorzugt, dass die Kügelchen kleiner sind, als die anzulagernden Zellen. Besonders bevorzugt sind die Kügelchen maximal nur ca. halb so groß wie der Durchschnitt der zu gewinnenden Zellen, und am meisten bevorzugt im Durchschnitt nur maximal ein Viertel so groß.

Bevorzugt wird das Verfahren unter Bedingungen durchgeführt, die es erlauben, dass sich die Zellen in intaktem Zustand an die Kügelchen anlagern, oder daran binden. Solche Bedingungen sind gegeben, wenn die Wechselwirkung der Zellen mit der Oberfläche der Kügelchen nicht gestört ist, bevorzugt, wenn die Zellen als Suspension in einem geeigneten Medium vorliegen, bevorzugt einem wässrigen Medium, wie z. B. Zellkulturmedium, Puffer mit einer für die betrachtete Zelle geeigneten Salzkonzentration, oder einer anderen wässrigen Lösung, die keinen negativen Einfluss auf die Zellen hat. Darüber hinaus sollte die Lösung/das Suspensionsmedium bevorzugt keine polaren Verbindungen in einer Menge enthalten, die die Wechselwirkung der Zellen mit den Oberflächen der eingesetzten magnetischen Kügelchen stört. Besonders bevorzugt liegen die Zellen in jeweils für die Zellen geeigneten Kulturmedien vor, oder in wässrigen Pufferlösungen, die eine für die jeweiligen Zellen nicht nachteilige Salzkonzentration haben. Diese Medien und Puffer sind Fachleuten auf diesem Gebiet bekannt und sind der üblichen Fachliteratur zu entnehmen. Beispiele für geeignete Medien sind DMEM, RPMI (jeweils mit oder ohne FCS und anderen Additiven), und als Waschpuffer PBS, jedoch können sämtliche übliche Zellkulturmedien für Säugetier- oder Insektenzellen verwendet werden und sämtliche. Puffer mit niedrigem bis mäßigem Salzgehalt.

Die Zellen werden mit den magnetischen Kügelchen ausreichend lange in Kontakt gebracht, d. h. über eine Zeitdauer, die ausreicht, um die Zellen an die Kügelchen binden/sich anlagern zu lassen. Eine solche Zeitdauer sollte mindestens 30 s, bevorzugt mindestens 1 min, weiter bevorzugt mindestens 3 min sein und kann beliebig lange z.B. über Nacht, oder bis zu 12 Std., bevorzugt bis zu 5 Std., besonders bevorzugt bis zu 30 min dauern.

Nach der Anlagerung/Bindung der Zellen an die magnetischen Partikel lassen sich die Zellen durch Anlegen eines Magnetfeldes an das Gefäß, in dem sich die Zellen mit den Kügelchen befinden, aus dem die Zellen umgebenden Medium sammeln, bzw. abtrennen. In einer bevorzugten Ausführungsform wird ein Magnet außen an das Gefäß angelegt, in dem sich die Zellen und die Magnetkügelchen befinden und das verbleibende Suspensionsmedium wird aus dem Gefäß abgegossen oder mit Hilfe einer geeigneten Vorrichtung entfernt, beispielsweise mit Hilfe einer Pipette abgezogen. Die Zellen können in einem geeigneten Waschmedium resuspendiert und dadurch gewaschen werden, indem wiederum ein Magnetfeld an das Gefäß angelegt wird und das Waschmedium wiederum aus dem Gefäß entfernt wird. Das vorliegende Verfahren stellt dadurch einen besonders schonenden Umgang mit den Zellen bereit, was die Qualität der Zellen für eine nachfolgende Behandlung, z. B. in Form einer Transfektion, begünstigt.

In einer bevorzugten Ausführungsform werden die gewonnenen und ggf. gereinigten Zellen anschließend einer Transfektion unterzogen, wobei die Zellen nicht von den magnetischen Kügelchen entfernt werden müssen, sondern vielmehr in einer besonders bevorzugten Ausführungsform an die Kügelchen gebunden/angelagert bleiben. Der Vorteil dieser Anlagerung an die Magnetkügelchen ist einerseits, dass kein weiterer Bearbeitungsschritt der Zellen notwendig ist, bevor eine Transfektion durchgeführt wird, andererseits wird die Transfektionsrate von Suspensionszellen nach Anlagerung an die gemäß dieser Erfindung einzusetzenden Kügelchen erhöht. Eine Theorie, an die die Erfindung nicht gebunden sein soll, warum eine solche Verbesserung der Transfektionsrate erreicht werden kann ist die, dass frei in Suspensionen vorkommende Zellen einzeln den Transfektionsverbindungen nur jeweils eine geringe Oberfläche darbieten, wohingegen die Zellen insgesamt in an die Kügelchen angelagertem Zustand eine größere Oberfläche bieten, indem sie in Form einer "vernetzten" Struktur vorliegen, bei der die Zellen über die dazwischenliegenden Kügelchen aneinandergelagert sind. Auf einer solchen Struktur bleibt das verwendete Transfektionssystem leichter haften (liegen), so dass insgesamt die erzielbare Transfektionsrate zunimmt.

Bevorzugte Transfektionssysteme für die Transfektion eukaryontischer Zellen sind solche, bei denen Transfektionsreagenzien zu den Zellen zugegeben werden, die dann über Endozytose in die Zellen aufgenommen werden. Diese Reagenzien können z.B. kationische Lipide, Dendrimere, Polyethylenimine, modifizierte Polyethylenimine und Mischungen daraus enthalten. Diese lagern sich an Nucleinsäuren (DNA; RNA in jeder zur Transfektion verwendeten und geeigneten Form) an und bilden so "Transfektionskomplexe". Diese liegen an den Zellen an und haften schwach an der Oberfläche, bis sie über Endozytose aufgenommen werden. Solche Transfektionsreagenzien sind auf dem Markt von verschiedenen Anbietern erhältlich. Bevorzugte Transfektionsreagenzien sind z.B. PolyFect®, Effectene®, Superfect® (alle zur Plasmidtransfektion; Qiagen, Deutschland); TransMessenger^{™} (RNA und siRNA Transfektion; Qiagen,Deutschland); RNAiFect^{™} oder HiPerFect (siRNA Transfektion; Qiagen, Deutschland).

Die Transfektionseffizienz kann insbesondere auch bei Zellen erhöht werden, die nur in geringer Menge, z. B. aus Blut oder Gewebe, isoliert werden können, und in Form von angelagerten Zellen an die magnetischen Kügelchen den Transfektionskomplexen deutlich konzentrierter "dargeboten" werden können.

Nach der Transfektion können die Zellen unmittelbar unter für die Zellen geeigneten Wachstumsbedingungen und -medien weiter kultiviert werden. Ein Entfernen der magnetischen Partikel ist nicht notwendig.

Die an die magnetischen Kügelchen gebundenen Zellen können einem Schritt zur Zell-Lyse unterworfen werden. Hierbei können entweder die Zellen unmittelbar nach dem Zellgewinnungsschritt, oder nach dem Aufreinigungsschritt, oder ggf. nach dem Transfektionsschritt (auch nach einer gewissen Zeitdauer der Kultivierung) lysiert werden. Die Lyse der Zellen findet durch Zugabe eines geeigneten Lysepuffers, bzw. durch Anlegen geeigneter Lysebedingungen statt, hierbei ist jede Bedingung und jedes Vorgehen geeignet, die zur Zellöffnung und somit zur Lyse der Zellen führt. Bevorzugt werden den Zellen lysierende Lösungen zugegeben, insbesondere Lösungen von Proteinase K und/oder Puffer geeigneter Salzkonzentrationen, oder die Zellen werden in deionisiertem Wasser aufgenommen, welches ggf. mit zusätzlichen lysierenden Komponenten versetzt ist. Geeignete lysierende Bedingungen für verschiedene Zelltypen sind Fachleuten auf diesem Gebiet bekannt und können der Literatur entnommen werden.

Die Lysebedingungen selbst sind kein limitierendes Merkmal für die vorliegende Erfindung, allerdings sind Lysebedingungen bevorzugt, die zu einer Lösung führen, aus der die durch Lyse freigesetzten Polynucleinsäuren unmittelbar an die magnetischen Kügelchen binden können, wie z.B. die Lyse bei hohen Salzkonzentrationen im Lysepuffer. In einem bevorzugten Verfahren gemäß der vorliegenden Erfindung können nämlich dieselben magnetischen Kügelchen, an die vor der Lyse die intakten Zellen gebunden/angelagert waren, unmittelbar nach der Lyse dazu dienen, die aus den Zellen freigesetzten Polynucleinsäuremoleküle zu binden. Durch Zugabe eines geeigneten Puffersystems, bzw. durch Anlegen geeigneter Bedingungen, lagern sich die frei gesetzten Polynucleotide an die in Suspension befindlichen magnetischen Kügelchen an. Die Bedingungen werden bevorzugt so gewählt, dass die Anlagerung der Nucleinsäuren gegenüber der Anlagerung der sonstigen Zellfragmente nach der Lyse bevorzugt wird.

Die an die magnetischen Kügelchen gebundenen Nucleinsäuremoleküle können aus dem Lysegemisch abgetrennt werden, indem wiederum ein Magnetfeld an das Gefäß, in dem sich die Kügelchen/die Nucleinsäuremoleküle befinden, angelegt wird.

Bevorzugte magnetische Kügelchen für den Einsatz in wenigstens einem, bevorzugt wenigstens zwei, besonders bevorzugt allen drei erfindungsgemäßen Teilschritten, die oben beschrieben sind, sind solche, die auf ihrer Oberfläche die folgenden Gruppen tragen: Glycin, 6-Aminocapronsäure, Asparaginsäure, NTA, PEI/PAA, PEI/PAA/NTA, HCl, Glycerin/Toluol, Glycerin/Diglyme, Pentaerithitol/Diglyme, Hydrazin, Asparaginsäure/Hydrazin, Bis-Tris/Diglyme oder DIPEA/Diglyme oder Kombinationen der vorgenannten. Auch geeignet sind NTA/NiSO₄, PEI/PAA/NTA/ NiSO₄, 6-Aminocapronsäure/NTA/ NiSO₄, PEI/Toluol/NTA/ NiSO₄, PEI/Diglyme/NTA/ NiSO₄, Polyethylenimin, Jeffamin 500 oder Bis-Tris/Toluol.

Das vorliegende Verfahren kann durch Einsetzen aufeinander abgestimmter Komponenten und Pufferlösungen durchgeführt werden, die in Form eines Kits bereitgestellt werden können. Beispielsweise kann ein solcher Kit magnetische Kügelchen enthalten, die für die Zellgewinnung, Zellaufreinigung und/oder Transfektion bestimmter Zellen angepasst sein können, ein Additiv, das die Lyse der Zellen bewirken kann und ggf. einen Aufreinigungspuffer für die Polynucleinsäureisolierung. In einer bevorzugten Ausführungsform enthält ein solcher Kit magnetische Kügelchen, die an ihrer Oberfläche eine schwach negative Gesamtladung aufweisen, wie z.B. durch Bindung von Carboxylatgruppen, einen Lysepuffer und einen Aufreinigungspuffer für Nucleinsäuremoleküle.

Ein Vorteil der erfindungsgemäßen Verfahrensschritte ist, dass während oder nach keinem der Schritte des Verfahrens ein Entfernen oder Wechseln der eingesetzten Kügelchen notwendig ist, sondern die eingesetzten Kügelchen in allen genannten Schritten, also Zellgewinnung, Zellaufreinigung, Transfektion, Lyse und Nukleinsäureaufreinigung (auch wenn sie unabhängig voneinander durchgeführt werden) dieselben sein können. Hierbei müssen auch nicht alle Schritte unter Einsatz der Kügelchen durchgeführt werden, beispielsweise kann die Zellgewinnung und/oder-reinigung noch ohne Kügelchen erfolgen und die Kügelchen erst ab dem Transfektionsschritt eingesetzt werden, jedoch ist ein Einsatz von der Zellgewinnung bis hin zu einer Transfektion und ggf. einer Lyse und Nukleinsäurereinigung bevorzugt. Ein weiterer Vorteil der erfindungsgemäßen Verfahrensschritte ist, dass jeweils keine Zentrifugationsschritte nötig sind, wodurch jeder der einzelnen Schritte vollständig automatisiert werden kann. Auch der Ablauf des gesamten Verfahrens von der Zellgewinnung bis zu RNA-Isolierung und Detektion kann vollständig automatisiert werden, da zu keinem Zeitpunkt die Kügelchen aus den Ansätzen entfernt werden müssen und ein Überführen der Gefäße, die die Zellen / Kügelchen enthalten, in eine externe Vorrichtung (z.B. eine Zentrifuge) entfällt. Somit kann das gesamte Verfahren in einer einzigen Vorrichtung durchgeführt werden, die wenigstens über eine Zugabemöglichkeit für Reagenzien (z.B. Pipettiervorrichtung) und wenigstens eine Vorrichtung zum Anlegen eines Magnetfeldes an ein Gefäß, das die Kügelchen enthält, verfügt. Auch eine solche Vorrichtung zur automatisierten Durchführung des erfindungsgemäßen Verfahrens ist daher Gegenstand der vorliegenden Erfindung.

Abbildungen:
Abbildung 1 zeigt die Ergebnisse der Cytotoxizitätstests gemäß Beispiel 2 mit HeLa Zellen.
Abbildung 2 zeigt die Ergebnisse der Cytotoxizitätstests gemäß Beispiel 2 mit Jurkat-Zellen.
Abbildungen 3 und 4 zeigen die Ausbeute an RNA, die sich aus an Beads gebundenen Zellengemäß Beispiel 3 isolieren lässt.
Abbildung 5 zeigt die Quantifizierung der RNA Ausbeute aus Zellen im Verhältnis zu der eingesetzten Bead-Konzentration.
Abbildung 6 zeigt die Ergebnisse der RNA Isolierung unter Einsatz von Bindungsadditiven gemäß Beispiel 4
Abbildungen 7 (A) (B) und (C) zeigen die Effizienz der Transfektion von Zellen unter Einsatz von Beads gemäß Beispiel 5

Die nachfolgenden Beispiele dienen lediglich der Darstellung der Erfindung und sollen diese nicht in irgendeiner Weise auf die dort gezeigten Ausführungsformen einschränken.

### Beispiele:

### Beispiel 1 Zellbindung magnetischer Kügelchen mit verschiedenen Oberflächenmodifikationen

Zu einer Kultur eukaryontischer Zellen (HeLaS3, Jurkat) mit einer Zelldichte von HelaS3 6x10⁴ , Jurkat 1.2x10⁵ / 500 ml wurde eine definierte Menge an magnetischen Kügelchen gegeben, die einem Zellen-zu-Kügelchen Verhältnis von 1 mg/ 500µl Zellsuspension entspricht.

Nach Inkubation für 10 min wurde die Bindung der Zellen an die Kügelchen unter dem Mikroskop betrachtet, bevor und nachdem ein magnetisches Feld an die Wand des Gefäßes angelegt wurde, in dem sich das Zell-/Kügelchengemisch befand. Tabelle 1 zeigt die Bindung der Zellen an die Kügelchen.

**Tabelle 1**

| **Name** | | **Modifikation** | **Zellbindung** |
|---|---|---|---|
| **RSC** | **001** | Glycin | ++ |
| **RSC** | **002** | 6-Aminocapronsäure | ++ |
| **RSC** | **003** | Asparaginsäure | ++ |
| **RSC** | **004** | NTA | ++ |
| **RSC** | **005** | PEI/PAA | ++ |
| **RSC** | **006** | PEI / PAA /NTA | ++ |
| **RSD** | **002** | HCI | ++ |
| **RSD** | **003** | Glycerin/Toluol | ++ |
| **RSD** | **004** | Glycerin/Diglyme | ++ |
| **RSD** | **005** | Pentaerythritol/Toluol | ++ |
| **RSD** | **006** | Pentaerythritol/Diglyme | ++ |
| | | | |
| **RSE** | **006** | Jeffamin 500 | + |
| **RSE** | **007** | Polyallylamin (20%) | +/- |
| **RSE** | **008** | Pentaethylen-hexamin | +/- |
| **RSE** | **012** | Polyethylenimin linear | + |
| **RSE** | **014** | Bis-Tris, Toluol | + |
| **RSE** | **015** | Bis-Tris, Diglyme | ++ |
| **RSE** | **017** | DIPEA, Diglyme | ++ |
| | | | |
| **RSH** | **001** | Hydrazin | ++ |
| **RSH** | **002** | Asparaginsäure/Hydrazin | ++ |
| **RSH** | **003** | PEI/PAA/NTA | ++ |
| **RSH** | **004** | PEI / PAA /NTA | ++ |
| | | | |
| **RSN** | **002** | NTA /NiSO4 | + |
| **RSN** | **003** | PEI / PAA /NTA / NiSO4 | + |
| **RSN** | **005** | PEI / PAA /NTA / NiSO4 | + |
| **RSN** | **006** | 6-Aminocaprons. / NTA / NiSO4 | + |
| **RSN** | **007** | PEI / PAA / NTA / NiSO4 | + |
| **RSN** | **008** | PEI / PAA / NTA / NiSO4 | + |
| **RSN** | **010** | PEI / Toluol / NTA /NiSO4 | + |
| **RSN** | **011** | PEI / Diglyme/ NTA / NiSO4 | + |

| | | | |
|---|---|---|---|
| ++ sehr gute Zellbindung, ohne wesentlichen Anteil freier Zellen + gute Bindung mit wenigen freien Zellen +/- mäßige Bindung mit deutlichem Anteil freier Zellen | | | |

### Beispiel 2

### Messung der Cytotoxizität von magnetischen Kügelchen in Zellkulturen

Die Toxizität der Kügelchen wird dadurch bestimmt, dass einer Zellkultur während der Inkubation in der Wachstumsphase der Zellen eine Menge von 0.004 g/ml der magnetischen Kügelchen zugegeben wird. Die Zellen werden unter geeigneten Wachstumsbedingungen in geeignetem Medium 24 h inkubiert und anschließend auf Cytotoxizität mit dem Roche LDH Cytotoxizitäts-Detektionskit gemäß den Anweisungen des Herstellers (Roche) untersucht. Hierfür werden die Kulturüberstände vor der Zelllyse gesammelt und die LDH (Lactatdehydrogenase) Aktivität gemessen. Die Menge an aus dem Cytosol zerstörter Zellen in das Medium abgegebenen Menge an LDH ist ein Maß für die Cytotoxizität von in der Kultur befindlichen Fremdstoffen.

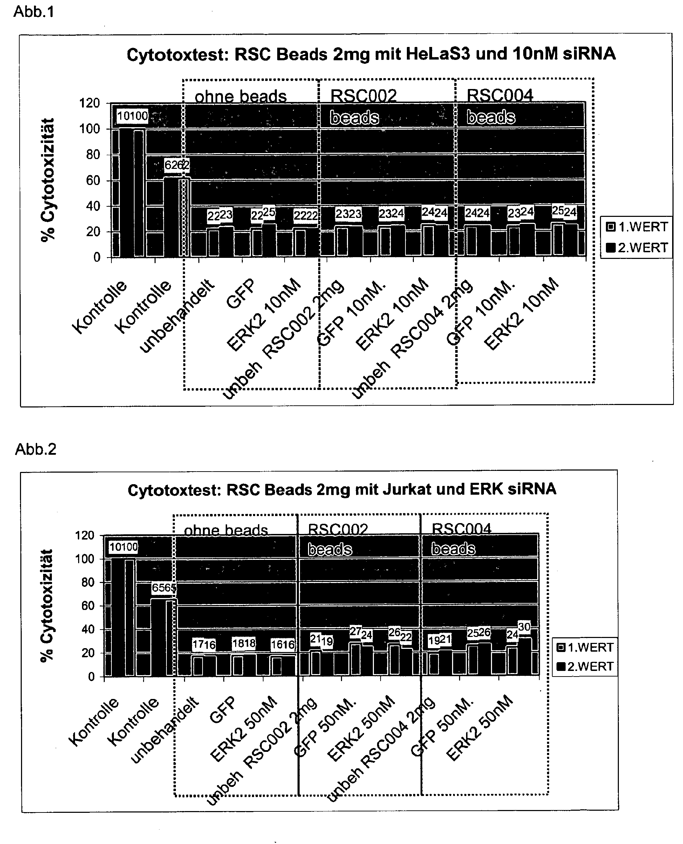

Wachstumsbedingungen: HeLaS3 Zellen wurden in DMEM (Dulbecco's modified Eagle Medium), komplementiert mit 10%FKS (fötales Kälber Serum) bei 37°C und 5% CO₂ in Luft inkubiert. Jurkat Zellen wurden in RPMI (Roswell Park Memorial Institute Medium), komplementiert mit 10%FKS (fötales Kälber Serum) bei 37°C und 5% CO₂ in Luft inkubiert. Die Ergebnisse der Cytotoxizitätstests sind in Abbildung 1 und 2 wiedergegeben.

### Beispiel 3

### Quantifizierung der Zellbindekapazität der magnetischen Kügelchen

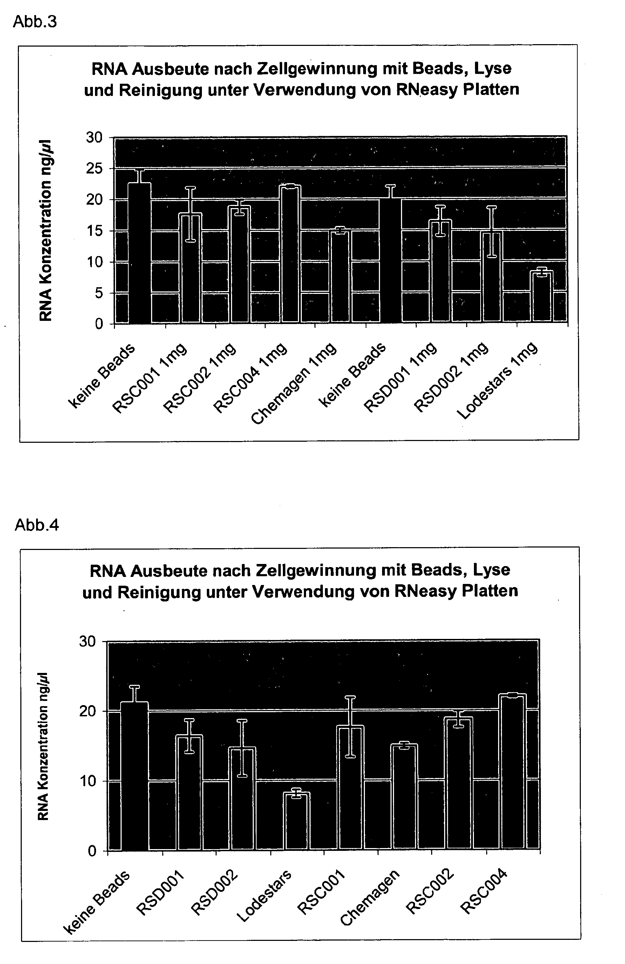

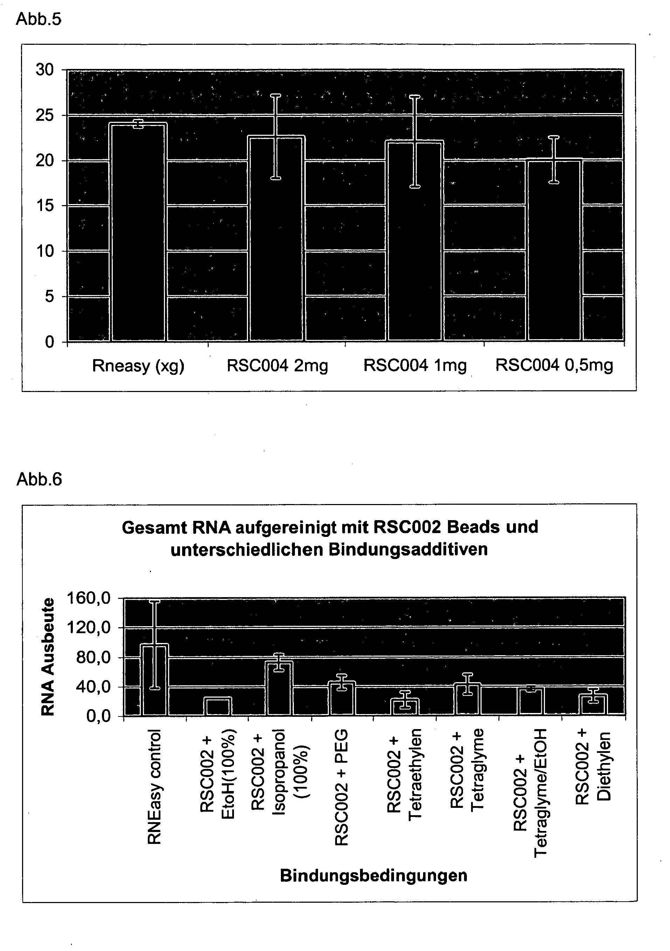

Die Zellbindekapazität der Kügelchen wird bestimmt, indem eine frische Zellkultur von Suspensionszellen in Aliquots gleicher Menge aufgeteilt wird, zu jedem Aliquot der Zellkultur jeweils 1 mg der magnetischen Kügelchen zugegeben wird, nach 10 min Inkubationszeit ein Magnetfeld angelegt wird und die Zellbindekapazität der Kügelchen über eine Gesamt-RNA Isolierung aus den gebundenen Zellen quantifiziert wird. Um die RNA-Isolierung unabhängig vom eingesetzten Kügelchentyp durchzuführen, wird die Isolierung mit Hilfe von RNEasy 96® Platten (Qiagen) und dem entsprechenden Isolierungsprotokoll durchgeführt. Als Kontrolle für die Zellmenge, die erhalten werden kann, werden die Zellen aus einem der Aliquots über Zentrifugation gewonnen und die RNA unter gleichen Bedingungen wie für die übrigen Proben isoliert. Die isolierte RNA wird spektrometrisch (Spectramax) bestimmt. Die Ergebnisse der erhaltenen RNA-Konzentrationen sind in den Abbildungen 3, 4 und 5 wiedergegeben. Die Bezeichnung "ohne Beads" stellt die Aufreinigung aus dem Kontrollansatz dar, bei dem die Zellen durch Zentrifugation gewonnen werden. Die Quantifizierung über die isolierte RNA stellt eine Relation zu der Zellbindekapazität der einzelnen untersuchten Kügelchen pro mg Kügelchen dar (Abbildung 3 und 4). In Abbildung 5 ist zu erkennen, dass eine weitere Erhöhung der Einsatzmenge der Kügelchen ab einem gewissen Anteil von Kügelchen zu Zellen die Ausbeute nicht weiter steigert.

### Beispiel 4

### RNA-Isolierung unter Einsatz von Bindungsadditiven

4*10⁵ K562 Zellen wurden mit 350µl RLT Puffer lysiert und mit 7.5 mg RSC002 Beads vermischt. Von Magattract® A und G Beads ist es bekannt, dass eine größere Menge an Beads für die gesamte RNA Aufreinigung nötig ist, als in den bisherigen Versuchen für die Bindung und Trasfektion der Zellen benutzt wurden. Um die Bindung an die Beads zu verbessern wurde nach Bindungsadditiven gescreened, für die aus bisherigen Arbeiten bekannt war, dass sie die Bindung an Beads verstärken. In Tabelle 2 ist eine Auswahl solcher Bindungsadditive gezeigt.

Zu dem Zell-lysat mit den Beads wurden jeweils 350µl Bindungsadditive gegeben und das Lysat wurde gut gemischt. Anschließend wurden die Beads/RNA 2x mit 500µl RWI Puffer und 2x mit 600µl RPE Puffer (Puffer aus dem RNEasy Handbook) gewaschen. Die Beads wurden anschließend für 10 min bei Raumtemperatur getrocknet und die Gesamt-RNA wurde mit 70µl RNAse-freiem Wasser eluiert. Die Gesamt-RNA wurde anschließend spektrometrisch quantifiziert. Die Ausbeute der erhaltenen RNA ist der folgenden Tabelle 3 zu entnehmen und ist in Abb.6 dargestellt.

**Tabelle 2**

| K562 Zellen und RSC002 Beads | |
|---|---|
| 1 | Kontrolle: Zellen, die durch Zentrifugation gewonnen wurden + RLT + EtOH (70%) |
| 2 | Zellen + RSC002 + RLT+ EtOH(100%) |
| 3 | Zellen + RSC002 + RLT + Isopropanol (100%) |
| 4 | Zellen + RSC002 + RLT + PEG 800 |
| 5 | Zellen + RSC002 + Tetraethylen (Tetraethylenglycol) |
| 6 | Zellen + RSC002 + RLT + Tetraglyme (Tetraethyleneglycoldimethylether) |
| 7 | Zellen + RSC002 + RLT + Tetraglyme/EtOH (73,5 %Tetraethyleneglycoldimethylether (257µl) und 24% EtOH (84µl)) |
| 8 | Zellen + RSC002 + RLT + Diethylen (Diethylenglycolmonoethyletheracetat) |

**Tabelle 3**

| Nr | Sample ID | Mean | SD |
|---|---|---|---|
| 1 | RNEasy control | 96,7 | 58,8 |
| 2 | RSC002 + EtoH(100%) | 23,7 | 0,1 |
| 3 | RSC002 + Isopropanol (100%) | 72,8 | 10,9 |
| 4 | RSC002 + PEG | 45,3 | 9,5 |
| 5 | RSC002 + Tetraethylen | 21,3 | 10,7 |
| 6 | RSC002 + Tetraglyme | 42,5 | 13,5 |
| 7 | RSC002 + Tetraglyme/EtOH | 36,6 | 3,1 |
| 8 | RSC002 + Diethylen | 26,6 | 8,9 |

→ Dieses Screening zeigt, dass die Bindebedingungen sehr gut optimierbar sind und dass die Beads auch für die RNA Aufreinigung geeignet sind.

### Beispiel 5

### Transfektionseffizienz

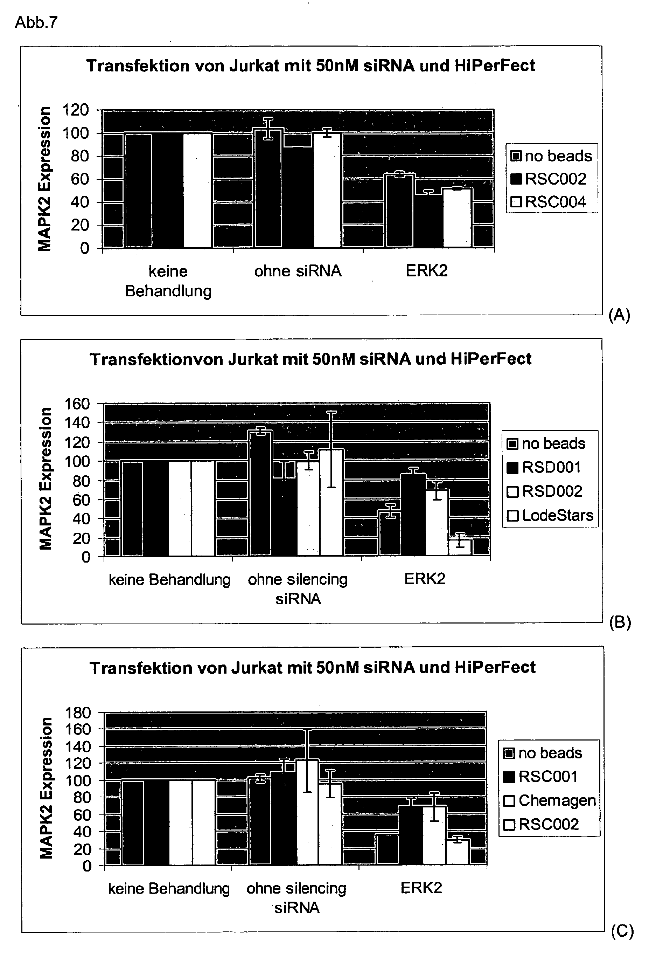

Die Wirksamkeit der Kügelchen auf die Transfektionseffizienz wurde durch Transfectionsexperimente mit Jurkat und HeLAS3 Zellen unter Verwendung des HiPerFect Transfektionsreagenz gemäß Anleitung des Herstellers (Qiagen) getestet, wobei jeweils 50nM MAPK2 siRNA (silencing RNA, bezeichnet als "ERK2") eingesetzt/transfiziert wurden. Als Kontrollen wurden einerseits Zellen ohne Anwesenheit von Kügelchen transfiziert, andererseits wurde das Transfektionsverfahren in Anwesenheit der Kügelchen ohne siRNA durchgeführt. Wie den Abbildungen 7 (A), (B) und (C) zu entnehmen ist, unterstützt die Anwesenheit der Kügelchen während der Transfektion die Aufnahme und steigert die Transfektionseffizienz, was sich in einer Verminderung der MAPK2-Expression durch "Silencing" dieses Gens auszeichnet. "Keine Behandlung" steht für die MAPK2-Expression vollständig unbehandelter Zellen.

## Patentansprüche

1. Verfahren zur Transfektion von Zellen, welches die Zugabe von magnetischen Kügelchen zu intakten Zellen umfasst, wobei die magnetischen Kügelchen eine Glas- oder Polymerbeschichtung umfassen und auf ihrer Oberfläche chemische Gruppen tragen, die eine unspezifische Bindung der Zellen an die Oberfläche erlaubt.

2. Verfahren nach Anspruch 1, wobei vor der Transfektion eine Zellaufreinigung und/oder Zellgewinnung mit Hilfe derselben Kügelchen erfolgt.

3. Verfahren gemäß Anspruch 1 oder 2, wobei die Zugabe der Kügelchen zu einer Zellsuspension erfolgt.

4. Verfahren gemäß einem der Ansprüche 1 bis 3, **dadurch gekennzeichnet, dass** es unter Bedingungen durchgeführt wird, die es erlauben, dass sich die Zellen in intaktem Zustand an die Kügelchen anlagern oder daran binden.

5. Verfahren gemäß einem der Ansprüche 1 bis 4, wobei nach der Anlagerung/Bindung der Zellen an die Kügelchen eine Transfektion der angelagerten/gebundenen Zellen mit Polynucleotidmolekülen erfolgt.

6. Verfahren zur Isolierung von Polynucleotiden aus zellulärem Material, umfassend ein Verfahren gemäß einem der Ansprüche 1 bis 5, wobei nach der Anlagerung/Bindung der Zellen an die Kügelchen und ggf. nach der Transfektion eine Lyse der angelagerten/gebundenen Zellen erfolgt.

7. Verfahren gemäß Anspruch 6, wobei nach der Lyse der Zellen eine Trennung der Polynucleotide von sonstigen zellulären Bestandteilen erfolgt.

8. Verfahren gemäß Anspruch 7, wobei die Trennung der Polynucleotide von den sonstigen zellulären Bestandteilen **dadurch** erfolgt, dass die Polynucleotide an die magnetischen Kügelchen angelagert/gebunden bleiben, während die sonstigen zellulären Bestandteile im Wesentlichen nicht an die Kügelchen binden.

9. Verfahren gemäß Anspruch 8, wobei die Trennung mit Hilfe eines Magneten erfolgt.

10. Verwendung von magnetischen Kügelchen, die mit einer Glas- oder Polymerbeschichtung überzogen sind und auf ihrer Oberfläche chemische Gruppen tragen, welche eine unspezifische Bindung der Zellen an die Oberfläche erlaubt zur Transfektion von Zellen.

11. Kit zum Durchführen eines Verfahrens gemäß einem der Ansprüche 1 bis 9, enthaltend magnetischen Kügelchen, die mit einer Glas- oder Polymerbeschichtung überzogen sind und auf ihrer Oberfläche chemische Gruppen tragen, welche eine unspezifische Bindung der Zellen an die Oberfläche erlaubt.

12. Vorrichtung zur Durchführung des Verfahrens gemäß einem der Ansprüche 1 bis 9, umfassend wenigstens eine Zugabevorrichtung und wenigstens eine Vorrichtung zum Anlegen eines Magnetfeldes an eine Gefäßwand.
